# EUROPEAN PATENT APPLICATION

(11) **EP 4 362 043 A1**
(43) Date of publication of application: **01.05.2024**
(21) Application number: 22204305.1
(22) Date of filing: 28.10.2022
(51) Int. Cl.: G16H 40/40, G16H 20/17

(54) **SOUND MONITORING METHOD FOR MONITORING A SOUND OF A MEDICAL DEVICE, A MEDICAL DEVICE WITH SOUND MONITORING AND A COMPUTER PROGRAM**

(71) Applicant: B. Braun Melsungen AG, 34212 Melsungen (DE)
(72) Inventor: BARTSCH, Stefan, 36286 Neuenstein (DE)
(74) Representative: Winter, Brandl - Partnerschaft mbB

(57) **Abstract**

The present disclosure relates to a computer-implemented sound monitoring method for monitoring a sound in a medical environment during an operation of a medical device (1), preferably a medical fluid pump (2), comprising the steps: Capturing the sound of the medical environment by a microphone (28) of a sound monitoring device (26) of the medical device (1); Input of the captured sound in an evaluation unit (30), preferably an Al-system (32) of the evaluation unit (30), especially preferred a classifier; Classifying of the captured sound by the evaluation unit (30) and assigning a predefined action/event to the respective captured sound; Determining by the evaluation unit (30) based on the classification, if the action is completed successfully or if an anomaly occurred in the operation of the medical device (1); and Displaying, in the event of an unsuccessful action and/or an anomaly an alarm or warning to a user by a displaying device (14). Besides, the present disclosure relates to a medical device, a computer program and to a computer-readable medium according to the subsidiary claims.

## Description

The present disclosure relates to a computer-implemented sound monitoring method for monitoring sound in a medical environment during an operation of a medical device. Further, the present disclosure relates to a medical device, a computer program, and a computer-readable medium according to the preambles of the subsidiary claims.

### Background of the present disclosure

Most medical devices require highly skilled and trained personnel to operate them correctly. Mistakes or faulty operation of the medical device due to wearing or incorrect use of the medical devices by medical personnel might result in serious harm to a patient treated with the medical device. Therefore, there is a need for monitoring the operation of the medical device.

Especially medical fluid pumps, such as infusion pumps and syringe pumps, have a high level of complexity and a number of mistakes or malfunctions can occur during the operation of the pumps due to errors in the handling of the devices. Examples of a malfunctioning or abnormal operation of the medical fluid pumps are for example clogging in tubes, a failing drive unit due to wearing in gears or bearings, or an incomplete insertion of tubes or syringes in the medical pumps. Detection of such malfunctions or abnormal operation and an alarm to a user would be advantageous.

One option for monitoring the medical devices would be to track any possible failure or abnormality by a set of sensors having for example different sensing technologies as well as different sensing locations. However, due to the sheer number of error sources, an unproportioned high number of sensors would be necessary for the fluid pumps. The high number of sensors would lead to further complexity in the control unit of the fluid pump and its software architecture and comes with further sources of errors. Therefore, there is a need for monitoring the operation of the medical devices using only a small number of sensors.

### Related prior art

For example, there exist speech controlled medical pumps that are known from the related art.

EP 3 975 203 A1 for example discloses a voice control of a healthcare facility. A medical device, for example, a hospital bed is capturing an audible command and is controlled by a voice control system. The voice control system comprises software for processing voice commands by a caregiver or a patient.

CN 1 07 742 519 A discloses a voice input system for controlling a medical pump. The voice input system captures the orders of a doctor or medical staff, recognizes an infusion plan, and sets the infusion pump such that the infusion plan is executed. The voice input system comprises voice recognition software. Therefore, the risk of incorrect inputs in the medical pump is reduced.

However, even though the related prior art provides a controlling interface, the related art does not provide any solutions for monitoring a correct operation of the medical device and thereby provide a safe therapy of a patient. In other words, in the prior art there is a lack of a monitoring of a safe and correct operation of the respective medical device.

### Brief description of the present disclosure

It is therefore an object of the present disclosure to overcome or at least mitigate the disadvantages of the prior art, and preferably provide a (cost-effective) method for monitoring the operation of a medical device, a medical device having a monitoring function as well as a computer program and a computer-readable medium. Preferably, a monitoring of the medical device may be conducted using only a single sensor. Further preferably, a single sensing technology may be adopted in order to monitor different kinds of operations of the medical device and evaluate them with regard to a correct action.

The object of the present disclosure is according to the present invention solved by a computer-implemented sound monitoring method in accordance with claim 1, with regard to a medical device in accordance with the features of claim 5, with regard to a computer program in accordance with the features of claim 14, and with regard to a computer-readable storage medium in accordance with the features of claim 15.

The present disclosure relates to a computer-implemented sound monitoring method for monitoring a sound in a medical environment before or during an operation of a medical device, preferably a medical fluid pump. The sound monitoring method comprises the following steps. A microphone (of a sound monitoring device of the medical device) captures the sound of the medical environment. This captured sound may include the sound of the medical device itself as well as the surrounding of the medical device. The captured sound is (digitally) inserted / inputted into an evaluation unit (of the sound monitoring device). The evaluation unit preferably comprises a trained Artificial Intelligence-system/ AI-system, especially preferred comprises a classifier. The evaluation unit classifies the captured sound and assigns a predefined action or event to the respective sound. The evaluation unit further determines based on the classification, if the action is completed successfully or if an anomaly occurred in the operation of the medical device. In the event of an unsuccessful action and / or an anomaly in the operation of the medical device, a displaying device (of the medical device) displays an alarm or warning to a user.

A general idea of the present disclosure is that a sound of a medical environment is, especially continuously, captured and thereby is monitored by the medical device. The captured sound is analyzed by the evaluation unit of the medical device. A (timeline section/snippet) of the captured sound may for example be extracted and isolated and this captured sound is first classified. For example, the captured sound is classified by the evaluation unit as a "closing of a door of the medical device". Within the classification of the closing of the door, a predefined action may for example be "properly closing the door". The evaluating unit is adapted to determine, especially by a trained Artificial Intelligence system that is trained with a data-set of sounds of closing the door of the medical device as input and "properly closed" as the output, whether the predefined action of "closing the door properly" is fulfilled correctly or not. In case of an anomaly of the closing of the door, a warning may be displayed or outputted. Therefore, with only a microphone, the medical device is capable of determining a correct operation of the medical device.

In an alternative example for illustration of the present subject-matter, where a medical device comprises a drip chamber, the evaluation unit may be adapted to classify the captured sound as a single drop in the drip chamber and further may be adapted to determine that there is a risk that the drip chamber may be overflowing when the count of drops exceeds a predefined number within a specified time limit.

The medical environment can for example be a room in which a patient is treated with the medical device. The microphone of the sound monitoring device could capture all sounds in the room; this might include sound emitted by the medical device as well as by the patient. Preferably, the sound monitoring device is able to capture sound from inside the medical device as well as sound from outside the medical device.

The sound of the operation of a medical device may be as well an audio track or audio signals that are captured by the at least one microphone. The audio signal as the captured sound may preferably be processed (electronically) before they are inserted into the evaluation unit.

The operation of the medical device may be for example the pumping of a fluid through a tube inserted into the medical device or the insertion of fluid via a syringe inserted into the medical device. A normal operation is to be understood as a pumping operation with the full functionality of the medical device. An anomaly in the operation of the medical device is to be understood as a malfunctioning of the medical device of any kind. Examples for such malfunctioning may be a broken gear in a drive unit of the medical device and/or an incomplete insertion of a syringe in the medical device.

The medical device can for example be an infusion pump or a syringe pump for inserting a medical fluid into a patient's body.

The evaluation unit can be a control unit having in particular a central processing unit (CPU). The evaluation unit might be a part of the sound monitoring device. However, a control unit of the medical device might also perform the function as the evaluation unit.

The predefined action is to be understood as a predefined event or mode of operation. Examples for an action/ an event are for example closing a lid of a medical fluid pump to fix the tube or syringe in the fluid pump.

The displaying device is preferably a displaying device of the medical device, preferably a (touch) display for displaying information about the medical device to the user.

In other words, the evaluation unit is adapted to evaluate the captured sound, especially based on a trained Artificial Intelligence system. The Artificial Intelligence system is preferably trained with regard to actions of the medical device. The evaluation unit evaluates the captured sound with regard to trained sound of the trained Artificial Intelligence system and determines the action based on the evaluated captured sound. The evaluation unit further determines, if the action is completed successfully or if an anomaly occurred in the operation of the medical device. The displaying device may display the result of the determination of the evaluation unit, preferably a confirmation message to the user in the event of a successful action. However, only in the event of an abnormal or unsuccessful action, the alarm or the warning may be displayed.

The sound monitoring device may be claimed independently as an isolated subject matter in a separate (divisional) patent application.

The sound monitoring method allows capturing the sound of the operation of the medical device and classifying the captured sound. Based on the classification of the captured sound, the evaluation unit can determine if an anomaly in the operation of the medical device occurred and warn the user if that is the case. Therefore, the sound monitoring method according to the present disclosure offers an easy and flexible method of monitoring the operation of the medical device. Furthermore, the sound monitoring method requires only one sensor, namely the microphone. Of course, alternatively more than one microphones may be provided in order to enhance a capturing of the sound or in order to locate the sound three dimensionally or in order to capture sound from the inside of the medical device as well as from the outside of the medical device. However, by using only one sensor capable of detecting a number of different errors or malfunctions, the sound monitoring method can be applied to a number of different fields of application.

The object of the present disclosure is further solved by a medical device, preferably a medical fluid pump, with a displaying device for visually displaying information to a user and a sound monitoring device for monitoring a sound in a medical environment during an operation of the medical device. The sound monitoring device comprises (at least one) a microphone for capturing a sound of the medical environment and an evaluation unit. The evaluation unit is adapted to classify the captured sound and assign a predefined action / event to the respective captured sound. The evaluation unit is further adapted to determine, if the action is completed successfully or if an anomaly occurred in the operation of the medical device. The displaying device displays in the event of an unsuccessful action and/or an anomaly an alarm or warning to a user.

The medical device according to the present disclosure allows monitoring of the operation of the medical device with only a single (acoustical) sensor. Therefore, the monitoring function is easy to implement in known medical devices without any substantial hardware or software changes.

Advantageous aspects of the present disclosure are claimed in the dependent claims and/or are in particular described herein below.

According to an optional feature of the present disclosure, the sound monitoring method further comprises training of the AI-system with a training data set comprising historical or previously recorded sounds of specific actions of the medical device as input values. The training data set further comprises a label with the respective action for the historical sounds as output values. Thereby, the AI-system may be specifically trained to the respective device and its predefined action of the medical device in advance in a controlled environment. For example in a sound studio, a closing of a lid of a infusion pump may be recorded several times with for example several velocities of closing the lid or with several different points of applying the closing force. These sounds together with the attribute/ label of "completely closing the lid" or "closing is correct" are captured as the input and output set of the training data. Further, in a controlled environment, a further training data set may be recorded with only half closing the lid or not completely closing the lid together with the attribute "anormal closing". Then the trained AI-system is specifically trained to detect a closing of the lid of the medical device and by its training data is capable of determining, whether the closing of the lid is "correct" or "incorrect" by analyzing a captured sound later on in the medical environment. This training can be done for other actions as well, such as for example whether the syringe bracket of the medical pump is closed completely.

Preferably, the method may further comprise the step of sending the result of the classification and determination of the action to a server, preferably to a cloud server and/or a remote database via WIFI.

Therefore, the AI-system is trained with a labeled training data set combining the previously recorded sounds of the specific actions and the action label for the respective sounds. The classifier is trained with the labeled training data set in a way that the trained classifier can take a sound as an input value and can assign an action label for the inserted sound as an output value. By doing this, the sound monitoring device can assign a label or an action to the captured sound.

Preferably, the AI-system is a neural network. Especially preferred, the AI-system is a neural network specialized on processing time-dependent data, for example convolutional neural networks or LSTM networks.

Preferably, the sound monitoring method according to the present disclosure includes the following steps. The following method is to be seen as an embodiment of the present disclosure and the present disclosure is not limited to the following example. In a first step, a latching sound of two infusion pumps latching onto each other is captured.

In a second step, the evaluation unit classifies the captured sound as latching of the two pumps and determines either as a complete latching or an incomplete latching of the two pumps. Based on the classification, the evaluation unit determines if the two pumps are latched together completely. If the evaluation unit determines that the pumps are not latched together, the displaying device of the medical device displays the alarm or warning to the user.

Preferably, the AI-system is adapted to be trained with a sound of a normal operation of the medical device and a sound of an abnormal operation of the medical device. The sound of the normal operation could be the sound of an electric motor and/or a gearbox of the medical device that is operation in its normal condition. The sound of the normal operation could also be the flowing sound of a liquid in a flexible tube. The sound of the abnormal operation would be any sound indicating wearing or another defect of the medical device. An example would be a scratching of a gear wheel and/or of a ball bearing. Thus, the AI-system can classify, if the captured sound belongs to a normal operation, or if the medical device might be defective.

Preferably, the AI-system is adapted to be trained with a sound indicating a vomitus and/or a sleeping behavior and/or a pain of a patient. The sound monitoring device might capture sound from inside and from outside of the medical device. Therefore, the sound monitoring device might capture sound emitted by a patient that is treated by the medical device. The AI-system might be trained with a training data set including human vomiting sounds and human sounds of pain. If the AI-system classifies captured sounds as pain or vomiting, the medical device might display an alarm to the user.

Preferably, the AI-system is (adapted to be) trained with a sound of a fluid flowing through a hose or a flexible tube. Fluid flowing through the flexible tube might indicate the normal operation of the medical device. A change of classification might indicate that a flow controller, preferably a roller clamp, on the flexible tube might have been opened or closed. Clogging of the tube might also be detected by the change of classification. The displaying device might display a respective disclaimer or warning. The tube might be clogged by the flow controller being closed for a long time. Therefore, the evaluation unit might determine if the flow controller is closed for a period longer than a predetermined time span and signal a respective warning to the user.

Preferably, the AI-system is (adapted to be) trained with a sound of a drop of fluid into a (drip) chamber. The classifier might be able to discover the sound of a fluid drop in the chamber. Preferably, the evaluation unit detects the sound of the drop as the action "dripping of a fluid". The evaluation unit might count every drip event or action discovered by the classifier and determine when the chamber is full. The displaying device might warn the user before the chamber is full.

Preferably, the AI-system is (adapted to be) trained with a sound of ventilation of the drip chamber. The displaying device might warn the user or issue a disclaimer to the user with the status of the ventilation. If the evaluation unit detects an unexpected stopping of the ventilation, the user can be warned.

Preferably, the AI-system is (adapted to be) trained with a sound of a fluid flowing through an open three-way stopcock. The evaluation unit might measure the time the stopcock is open and determine a volume of the fluid that passed the stopcock. The evaluation unit might also be able to discover a clogging of the stopcock in case of a switch in classification.

Preferably, the AI-system is (adapted to be) trained with a sound of a complete latching of multiple medical devices against each other and/or with a station and a sound of an incomplete latching of the multiple medical devices and/or the station. A warning can be issued to the user if the medical devices are not properly latched to each other or the station.

Preferably, the AI-system is (adapted to be) trained with a sound of a clamp, preferably a ratch of a latching connector, indicating whether the clamp closed completely around a pole. A warning can be issued to the user if the medical devices are not fixed at the pole with the clamp.

Preferably, to sum up, the captured sound could be matched to one or a combination the following actions:
- Flow of liquid in the flexible tube;
- Closing the lid of the medical pump;
- Closing the claws of the syringe pump;
- Closing the syringe bracket of the syringe pump;
- Drop of fluid in the drip chamber of the medical device;
- Ventilation of the drip chamber;
- Flow of liquid in the stopcock;
- Latching of medical devices against each other or against the station; and/or
- Closing of a clamp for fixating the medical device or the station on a pole.

According to an independent aspect of the present disclosure that may be claimed independently as a separate subject-matter, preferably in a separate patent application, the objective is solved by a medical device, preferably a medical fluid pump, (optionally having a displaying device for visually displaying information to a user) and a sound monitoring device for monitoring a sound in a medical environment during an operation of a medical device, the medical device comprising a housing having an opening (between the inner and the outer side of the housing), the sound monitoring device being arranged in the inner side of the housing and comprising a microphone being in (fluid) connection with the opening of the housing for capturing sound from inside the housing and from outside the housing. Preferably, the medical device comprises a housing with an opening. The sound monitoring device may be arranged at an inner side of the housing and might comprise the microphone that is in (fluid) connection with the opening of the housing for capturing sound from inside the housing and from outside the housing.

The inner side of the housing is to be understood as the side of the housing with for example the power unit and the motor of the medical device. The microphone can capture sound from the inside of the housing, especially the sound of the power unit, such as the gears, bearings, and/or the electric motor of the device. Through the opening, the microphone might further capture sound from outside the housing, especially from the medical environment around the medical device. Therefore, the microphone is able to capture the sound of the entire medical environment during the operation of the medical device.

Preferably, the sound monitoring device comprises a printed circuit board/ PCB on which the microphone is arranged, the PCB having a PCB-opening that is arranged in extension to the opening of the housing in order to provide a fluid connection between the microphone and the opening of the housing. The PCB-opening might allow the microphone to capture sound from outside the housing.

Preferably, the medical device comprises a membrane on the outer side of the housing in the area of the opening, in order to seal / cover / barrier the opening of the housing towards the outer side. However, the membrane is capable to transmit a sound from the outer side to the microphone.

Preferably, between the PCB and the housing, a circumferentially closed gasket is arranged in extension to the opening of the housing and to the opening of the PCB in order to improve sound transmission to the microphone and to block other ambient noises.

Preferably, the medical device comprises a storage unit for storing unknown sounds. If a sound is classified as unknown during the classification of the captured sound, the medical device might display a warning to the user and might invite the user to label the sound. The user might input the label via the displaying device. If the unknown sound is labeled by the user, the sound with the label can be added to a sound database and can be used to train the AI-system and increase the model accuracy.

According to one embodiment, the microphone may be a MEMS microphone.

According to an alternative embodiment, the opening of the housing as well as the PCB-opening may have a circular shape and is forming a cavity, wherein the diameter of the opening of the housing is minimum 2x and/or maximum 4x the diameter of the PCB-opening.

The present disclosure further relates to a computer program comprising instructions, which, when the program is executed by a computer, cause the computer to carry out the method of one of the previous aspects.

The object of the present disclosure is further solved by a computer-readable medium comprising instructions that when executed by a computer, cause the computer to carry out the method of one of the previous aspects.

Preferably, the computer-implemented sound monitoring method may further comprise the steps of evaluating an ambient sound level (amplitude) of the captured sound, and adjusting a volume of an issued audible alarm accordingly. Preferably, (analogue to the method) the evaluation unit of the medical device may be adapted to evaluate the ambient sound level of the captured sound of the medical environment and to adjust a volume of an audible alarm of the medical device accordingly. For example, if the sound level is evaluated high, the volume is adjusted high as well. By using the sound detector (sound monitoring device), preferably with an AI-System, it is also possible to capture and evaluate the ambient sound level (amplitude) in, for example, a patient's room or a medical operating room and dynamically adjust the volume of the (audible) alarms issued accordingly. This would lead to a decrease in the sound level in the room or on the ward and, conform to various studies that say that a higher sound level has a negative impact on patient recovery in the long term, would in the end benefit the patient.

The disclosure with regard to the sound monitoring method according to the present disclosure applies analogue to the medical device according to the present disclosure as well as vice versa.

### Brief description of the figures

The present disclosure is explained in more detail below with reference to preferred embodiments with the aid of figures.
- Fig. 1: shows a medical device according to a first embodiment of the present disclosure,
- Fig. 2: shows a medical device according to a second embodiment of the present disclosure,
- Fig. 3: shows a schematic view of the medical device according to an embodiment of the present disclosure,
- Fig. 4: shows a flow chart of a sound monitoring method according to an embodiment of the present disclosure,
- Fig. 5: shows a schematic view of the sound monitoring device and the medical device,
- Fig. 6: shows a perspective view of an upper housing shell of the medical device according to an embodiment of the present disclosure, and
- Fig. 7: shows a flow chart of a sound monitoring method according to an embodiment of the present disclosure.

The figures are schematic in nature and are intended only to aid understanding of the invention. Identical elements are provided with the same reference signs. The features of the various embodiments can be interchanged.

### Detailed description of preferred embodiments

In the following, examples of the present disclosure will be described in detail using the accompanying figures.

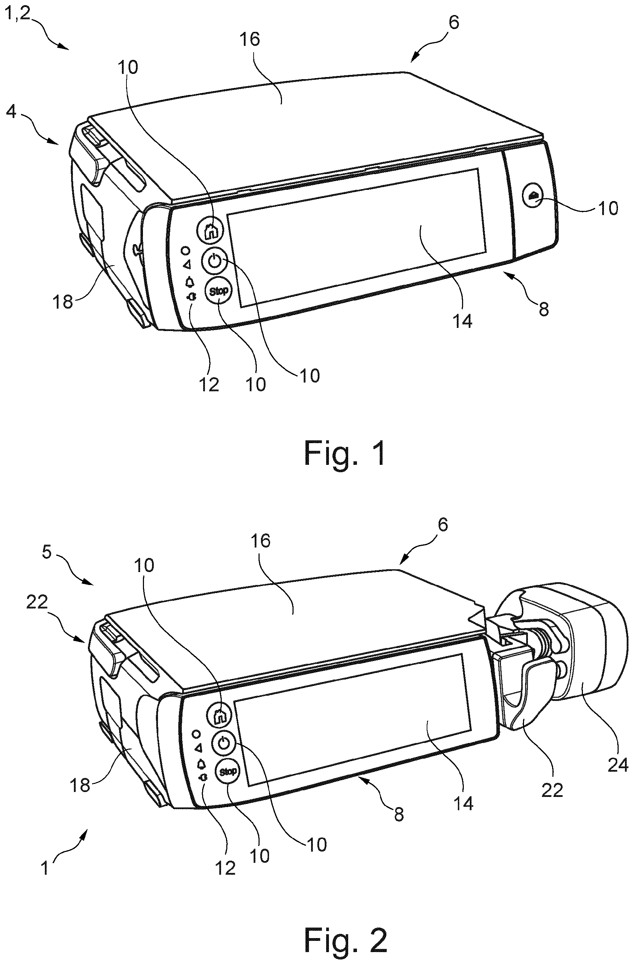

Fig. 1 shows a medical device 1, preferably a medical fluid pump 2, in accordance with a preferred embodiment of the present disclosure. The fluid pump 2 illustrated in Fig. 1 is designed as an infusion pump 4. It is to be understood that the fluid pump 2 in accordance with the present disclosure may also be designed as a syringe pump 5 (shown in Fig. 2).

As illustrated in Fig. 1, the medical fluid pump 2 comprises a substantially cuboid pump housing 6 to the front side of which a front lid 8 is pivotably hinged. A plurality of operating keys 10, a plurality of signal lights 12, and a displaying device 14, preferably an (integrated rectangular) touch display, are arranged on a front side of the front lid 8.

The pump housing 6 further comprises a housing upper shell 16 and a housing lower part/housing lower shell 18 which are preferably adapted to be detachably connected with each other and thus form the pump housing 6. A carrying handle 20 formed as a U-shaped handle bracket is pivotably hinged to the housing upper shell 16. The lid 8 can be folded about an axis to insert a (flexible) tube into the fluid pump.

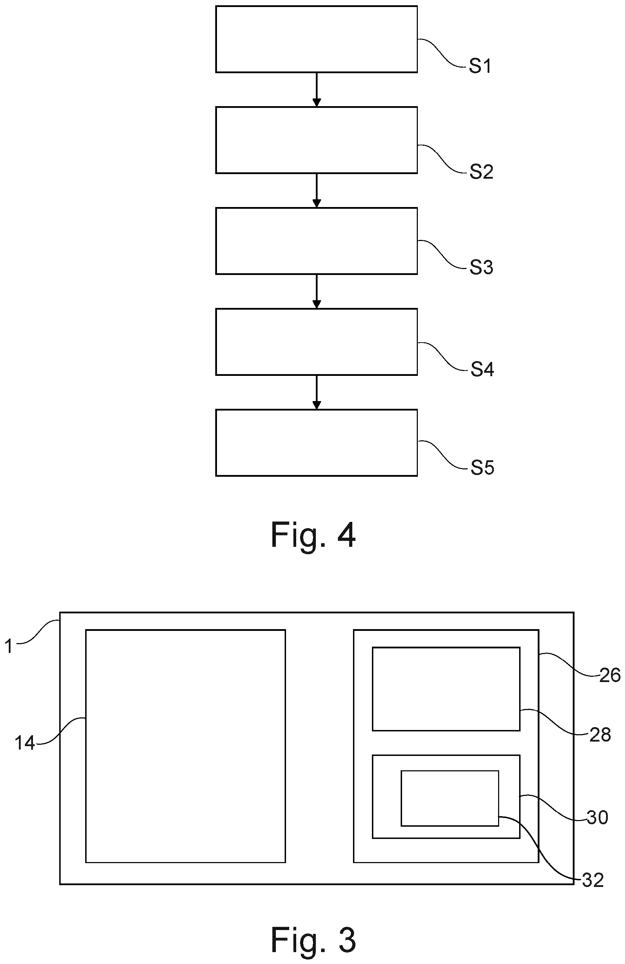

As explained later in detail with regard to Fig. 3, in contrast to the prior art, the medical device 1 comprises a specific sound monitoring device 26 that is adapted to monitor the sound and thereby monitor the correct operation of the medical device 1.

Fig. 2 shows another embodiment of a medical device 1 according to the present disclosure in the form of a syringe pump 5. The syringe pump 5 also comprises a substantially cuboid pump housing 6 with the front lid 8 and the integrated touch display 14. The syringe pump 5 has on one of its side surfaces (on the right in this front view) a syringe bracket 22 as well as a drive head 24, with the aid of which a syringe (not shown) can be attached and compressed with a linear movement of the drive head 24 in the direction of the pump housing 6. The pump housing 6 of the syringe pump 5 has a recess on one side due to the drive head 24 and the syringe bracket 22, i.e. the housing 6 is designed in such a way that the drive head 24 including the syringe bracket 22 can be attached to it. To insert the syringe, the front lid 8 is first folded about an axis on the lower side of the front lid 8 in the direction of the floor and the syringe bracket 22, which is fitted between the displaying device 14 and the drive head 24, is pulled out and then rotated through 90°, exposing the mounting location of the syringe. Clamping the syringe into / onto the drive head 24, locking it via the syringe bracket 22, and closing the front lid 8 completes the process of mounting the syringe into the syringe pump 5.

Opening the front lid 8 is enabled via a recess. The front lid 8 comprises a plurality of operating keys 10 and a plurality of signal lights 12 for the syringe pump 5. Similarly to the first embodiment of Fig. 1, the syringe pump 5 comprises as well a sound monitoring device 26 that is adapted to monitor the sound and thereby monitor the correct operation of the medical device 1 and will be described in the following with regard to Fig. 3.

Fig. 3 shows a general schematic view of the medical device 1 in order to illustrate the specific configuration of the sound monitoring device 26. The medical device 1 comprises the displaying device 14 and the sound monitoring device 26 for monitoring a sound in a medical environment during an operation of the medical device 1. The sound monitoring device 26 comprises a microphone 28 (as a single sensor) for capturing a sound of the medical environment and an evaluation unit 30. Alternatively, in another embodiment (not shown) the sound monitoring device 26 may comprise not only one single microphone, but also several microphones in order to enable the sound monitoring device to localize a sound for example. By this configuration, the medical device is capable to detect and capture a sound of the medical environment by the microphone 28, for example one single MEMS microphone, and provide the captured sound as digital data to the evaluation unit 30. The evaluation unit 30 in this embodiment comprises an AI-system 32, especially preferred a classifier, that is adapted for classifying the captured sounds. The AI-system 32 is trained with specific actions and whether these actions are executed correctly or incorrectly, such for example a correct and/or incorrect closure of front lid 8 of the medical device 1 of Fig. 1 or a closure of a syringe bracket 22 of Fig. 2. The evaluation unit 30 is further adapted to determine, if the action is completed successfully or if an anomaly occurred in the operation of the medical device 1. Thereby the sound monitoring device is capable of monitoring an safe operation of the medical device. For example, before the therapy of the patient, the sound monitoring device may be trained with a specific insertion of a syringe and a closing of the bracket. Then, when for example a medical personal is inserting the syringe with a front side tube, the sound monitoring device 26 may control the insertion steps of the syringe and may determine, whether the syringe is first inserted correctly, second the syringe bracket is closed properly / correct and third the tube is inserted in its predefined position. After the successful insertion, the display device is outputting a message to the user, that the syringe is inserted correctly. Otherwise, when for example the syringe bracket is not closed properly, the medical device may output via the displaying device a warning to a user and interrupt a function of the medical device.

In another example, during an operation of a fluid pump for example, the sound monitoring device 26 may capture a sound of the pump and may classify and determine by the AI-system 32, that a malfunction of the pump has occurred and output a warning message to a user.

With the help of the sound monitoring device 26, a correct operation of a medical device 1 can be monitored and a safety of a patient may be increased.

Fig. 4 shows a flow chart of a sound monitoring method according to a preferred embodiment for monitoring a sound in the medical environment during the operation of the medical device 1. In step S1, the microphone 28 captures the sound of the medical environment. The sound of the medical environment includes a sound from the outside of the medical device 1 as well as a sound from the inside of the medical device 1. The captured sound is inserted in the evaluation unit 30 in step S2. In step S3, the evaluation unit 30 that has in this embodiment the AI-system 32, classifies the captured sound and assigns a predefined action/event to the respective captured sound, such as a closing of the lid or the closing of a syringe bracket. The evaluation unit 30 determines, based on the classification, if the action is completed successfully or if an anomaly occurred in the operation of the medical device 1.

In step S5, in the event of an unsuccessful action and/or an anomaly the displaying device 14 displays an alarm or warning to the user.

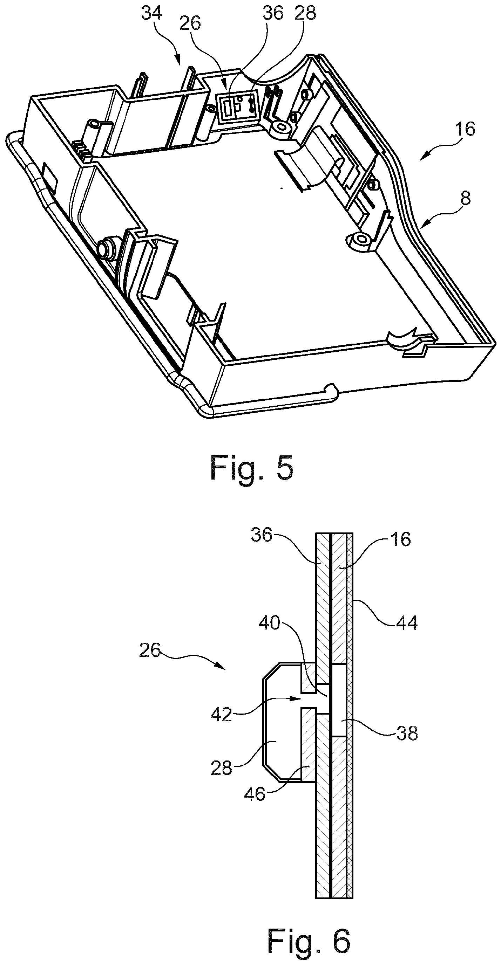

Fig. 5 shows a perspective view of the upper housing shell 16 of the medical device 1. The upper housing shell 16 is a substantially flat top and four sidewalls that extend vertically from the top. The four sidewalls 34 comprise one front cover with the front lid 8 and the displaying unit 14 and one rear side. The sound monitoring device 26 is positioned at one of the sidewalls, preferably at the left sidewall when looking at the medical device 1 from the front. The sound monitoring device 26 comprises a printed circuit board/ PCB 36 and the microphone 28. The sound monitoring device 26 is arranged to have a (larger) distance to an electric motor (not shown) of the fluid pump 2 so that the noise of the motor does not superimpose the sound outside the pump housing 6. If the sound monitoring device 26 is attached to the syringe pump, the sound monitoring device 26 is preferably attached to the opposite side of the housing 6 as the drive head 24.

Fig. 6 shows a schematic view of the sound monitoring device 26 on the housing of the medical device 1. The sound monitoring device 26 comprises the PCB 36 that is attached to the pump housing 6, preferably to the upper housing shell 16, of the medical device 1. The pump housing 6 has an opening 38 that extends through the thickness of the housing shell. The PCB 36 comprises a PCB-opening 40 that is arranged towards the opening 38 of the pump housing 6 to form a common opening. The microphone 28 of the sound monitoring device 26 is attached to the PCB 36 and comprises a sound port 42 that is in connection with the PCB-opening 40 for the sound to be transported through. Thus, the microphone 28 is in fluid connection with the opening 38 of the housing shell. On the outer side of the housing shell is a membrane 44. The membrane 44 covers the opening 38, such that the opening 38 forms a cavity with air inside. Sound from outside the pump housing 6 causes vibration of the membrane 44. The membrane 44 transfers the vibration via the air in the cavity to the microphone 28. Preferably, the sound monitoring device 26 comprises a gasket 46 between the PCB 36 and the microphone 28 to improve the capturing of sound from outside the pump housing 6.By this configuration, the sound monitoring device 26 is sealed against the outside but is enabled to still capture sound from the outside of the housing. Further, it can be cost efficiently be produced and arranged on the housing of the medical device 1 while maintaining the advantage of being modular and compact in size.

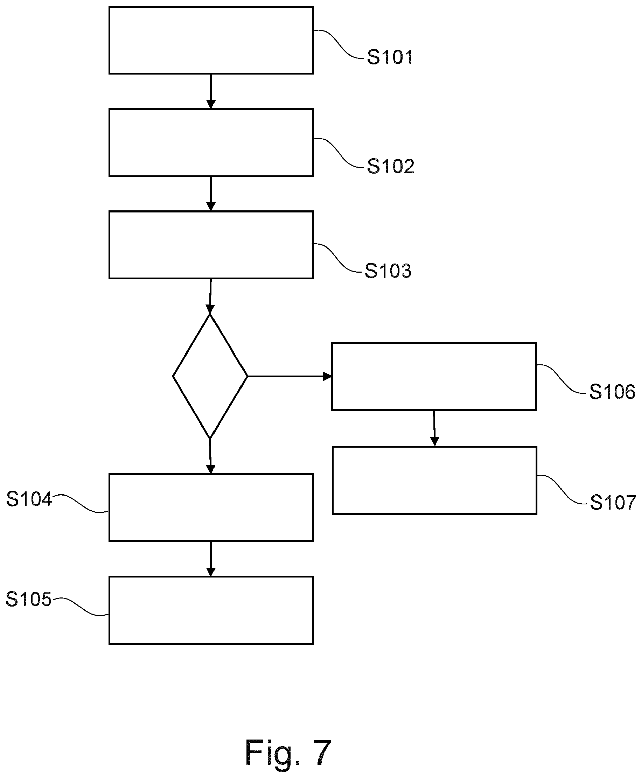

Fig. 7 shows a flow chart of a sound monitoring method according to another embodiment. Steps S101 and S102 correspond to steps S1 and S2 in Fig. 4. In step S103 the evaluation unit 30 classifies the captured sound. Either the captured sound is known to the evaluation unit 30 because it was in the training data set of the AI-system 32, or the captured sound is unknown. If the captured sound is classified as a known sound and the evaluation unit 30 was able to assign an action to the sound, the evaluation unit 30 determines in step S104, if the action was completed successfully. In step S105 a warning is outputted to the user, if the action was not completed successfully.

However, if the evaluation unit 30 classifies the captured sound as an unknown sound, the evaluation unit 30 warns the user that an unknown sound was captured in step S106 and/or requests in step S106 the user to label the unknown sound. The user can input a label or the action for the unknown captured sound. If the user labels the captured sound, the sound can be added to a database stored on a storage unit of the medical device in step S107. If the database is expanded during the operation of the medical device, the model of the AI-system can be enhanced.

### List of reference signs

- 1: medical device
- 2: medical fluid pump
- 4: infusion pump
- 5: syringe pump
- 6: housing
- 8: front lid
- 10: operating keys
- 12: signal lights
- 14: displaying device
- 16: upper housing shell
- 18: lower housing shell
- 20: Carrying handle
- 22: syringe bracket
- 24: drive head
- 26: sound monitoring device
- 28: microphone
- 30: evaluation unit
- 32: AI-system
- 34: sidewall
- 36: PCB
- 38: opening
- 40: PCB-opening
- 42: sound port
- 44: membrane
- 46: gasket

- S1; S101: Step capturing sound
- S2; S102: Step input
- S3; S103: Step classifying captured sound
- S4; S104: Step determining if action is successful or abnormal
- S5; S105: Step displaying
- S106: Step warning of unknown sound
- S107: Step storing labled unknown sound in database

## Claims

1. A computer-implemented sound monitoring method for monitoring a sound in a medical environment before or during an operation of a medical device (1), preferably a medical fluid pump (2), **characterized by** the steps:
- Capturing a sound (S1; S101) of the medical environment by a microphone (28) of a sound monitoring device (26) of the medical device (1);
- Input (S2; S102) the captured sound into an evaluation unit (30), preferably into an trained AI-system (32) of the evaluation unit (30), especially preferred into a classifier;
- Evaluating and classifying (S3; S103) of the captured sound by the evaluation unit (30) and assigning a predefined action to the respective captured sound based on the classification;
- Evaluating and determining (S4; S104) by the evaluation unit (30) based on the classification, if the action is completed successfully or if an anomaly occurred in the operation of the medical device (1);
- Displaying (S5; S105), in the event of an unsuccessful action and/or an anomaly an alarm or warning to a user by a displaying device (14), preferably a message with respect to the unsuccessful action, and further preferably, in the event of a successful action, displaying a confirmation message to a user by the displaying device (14).

2. The computer-implemented sound monitoring method according to claim 1, **characterized in that** the sound monitoring method further comprises training of the evaluation unit (30) with a training data set comprising historical sounds of specific operations of the medical device (1) as input values and a label with the action for the respective historical sounds as output values, especially before the step of capturing the sound of the medical environment.

3. The computer-implemented sound monitoring method according to claim 1 or 2, **characterized in that** the step capturing the sound of the medical environment and the step classifying of the captured sound is performed with regard to a safe closure of a specific latching unit of the medical device (1), and the determined action is whether the locking unit is safely closed, and in the event of an determination of an unsafe closure of the locking unit, the step displaying comprises a displaying a warning message that the locking unit is not locked and unsafe and preferably requests an input by a user to confirm the warning.

4. The computer-implemented sound monitoring method according to claim 2, **characterized in that,** if the captured sound is classified as an unknown sound by the evaluation unit (30), the evaluation unit (30) requests the user (S106) to label the unknown captured sound via an input device, preferably via a touch display, and, if the unknown captured sound is labeled by the user, the labeled sound is added (S107) to a storage unit by the evaluation unit (30).

5. A medical device (1), preferably a medical fluid pump (2), having a displaying device (14) for visually displaying information to a user and a sound monitoring device (26) for monitoring a sound in a medical environment before or during an operation of the medical device (1) comprising a microphone (28) for capturing a sound of the medical environment, and an evaluation unit (30) that is adapted to:
- Evaluate and classify the captured sound, preferably based on a trained AI-system (32) of the evaluation unit (30), and assign a predefined action to the respective captured sound,
- Evaluate and determine, if the action is completed successfully or if an anomaly occurred in the operation of the medical device (1),
- Displaying, in the event of an unsuccessful action and/or an anomaly, an alarm or warning to a user by the displaying device (14), preferably a message with respect to the unsuccessful action, and further preferably, in the event of a successful action, displaying a confirmation message to a user by the displaying device (14).

6. The medical device (1) according to claim 5, **characterized in that** the evaluation unit (30) comprises the trained AI-system (32) that is trained with at least one sound of a normal operation of the medical device (1), preferably a sequence of sounds of a normal operation, and/or at least one sound of an abnormal operation of the medical device (1).

7. The medical device (1) according to any one of claims 5 to 6, **characterized in that** the AI-system (32) is trained with
a sound of a complete latching of multiple medical devices (1) with each other and/or with a station, and/or
a sound of an incomplete latching of the multiple medical devices (1) and/or the station.

8. The medical device (1) according to any one of claims 5 to 7, **characterized in that** the medical device (1) is a medical fluid pump (2) and the AI-system (32) is trained with a sound of a syringe bracket (22) indicating whether the syringe bracket (22) is closed completely around a syringe, preferably a sound of a completed closure of the syringe bracket (22), and further preferably as well as a sound of an uncompleted closure of the syringe bracket (22).

9. The medical device (1) according to any one of claims 5 to 8, **characterized in that** the medical device (1) is a medical fluid pump (2) and the AI-system (32) is trained with a sound of a front lid (8) indicating whether the front lid (8) closed completely around a hose or flexible tube inserted into the medical fluid pump (2).

10. The medical device (1) according to any one of claims 5 to 9, **characterized in that** the AI-system (32) is trained with a sound indicating that wear on the medical device (1) occurred, preferably a scratching of a gear wheel and/or of a ball bearing.

11. The medical device (1) according to any one of claims 5 to 10, **characterized by** a housing (6) having an opening (38), the sound monitoring device (26) being arranged in the inner side of the housing (6) and comprising a microphone (28) being in connection with the opening (38) for capturing sound from inside the housing (6) and from outside the housing (6).

12. The medical device (1) according to claim 11, **characterized in that** the sound monitoring device (26) comprises a PCB (36) on which the microphone (28) is arranged, the PCB (36) having a PCB-opening (40) that is arranged in extension to the opening (38) of the housing (6) in order to provide a fluid connection between the microphone (28) and the opening (38).

13. The medical device (1) according to claim 11 or 12, **characterized in that** on the outer side of the housing (6) in the area of the opening (38), the medical device (1) comprises a membrane (44) in order to seal the opening (38) towards the outer side but to transmit a sound to the microphone (28).

14. A computer program comprising instructions, which, when the program is executed by a computer, cause the computer to carry out the method of one of the claims 1 to 4.

15. A computer-readable medium comprising instructions which, when executed by a computer, cause the computer to carry out the method of one of the claims 1 to 4.
